# FASCICULE DE BREVET EUROPEEN

(11) **EP 2 115 420 B1**
(45) Date de publication et mention de la délivrance du brevet: **05.10.2016**
(21) Numéro de dépôt: 07803829.6
(22) Date de dépôt: 03.07.2007
(51) Int. Cl.: G01N 1/22, G01N 33/00, G01N 1/10

(54) **DISPOSITIF DE PRELEVEMENT AUTOMATIQUE DU TRITIUM DANS LA VAPEUR D'EAU DE L'AIR**
VORRICHTUNG FÜR DIE AUTOMATISCHE ENTNAHME VON TRITIUM AUS WASSERDAMPF IN DER LUFT
DEVICE FOR AUTOMATICALLY SAMPLING TRITIUM IN THE WATER VAPOUR IN AIR

(30) Priorité: 04.07.2006 FR 0606065
(43) Date de publication de la demande: 11.11.2009
(73) Titulaire: Etat français représenté par le Délégué Général pour l'Armement, 75509 Paris Cedex 15 (FR); Institut de Radioprotection et de Sûreté Nucléaire, 92140 Clamart (FR)
(72) Inventeur: BARON, Yves, 50100 Cherbourg Octeville (FR); MARO, Denis, 50100 Cherbourg Octeville (FR)
(86) Numéro de dépôt international: PCT/FR2007/001121
(87) Numéro de publication internationale: WO 2008/003853

(56) Documents cités:
- FR-A1- 2 528 321
- JP-A- 11 064 532
- JP-A- 2001 330 695
- JP-A- 2005 016 980
- US-A- 3 712 074
- US-A- 4 638 674

## Description

L'invention concerne la mesure de la concentration en tritium de la vapeur d'eau de l'air. Plus précisément, l'invention a trait à un dispositif automatique de prélèvement du tritium dans la vapeur d'eau de l'air.

On sait que les prélèvements de vapeur d'eau tritiée peuvent être réalisés par piégeage de la vapeur d'eau de l'air par différents moyens tels que barboteur, agents de dessiccation ou pièges froids.

Les systèmes de prélèvement par barboteur dans des biberons contenant de l'eau des Abatilles présentent l'inconvénient de nécessiter des temps de prélèvement importants, de l'ordre de plusieurs jours, du fait d'un faible débit de prélèvement et d'une dilution du tritium dans les biberons d'eau des Abatilles.

Un autre moyen de prélèvement consiste à faire passer de l'air dans des supports contenant des agents de dessiccation, tels que les tamis moléculaires ou les gels de silice. La vapeur d'eau est retenue dans ces supports pourvus d'une grande surface spécifique qui leur confère un fort pouvoir d'adsorption vis-à-vis des petites molécules polaires. Afin de récupérer l'eau pour le dosage du tritium, il est nécessaire d'effectuer un cycle d'évaporation et de condensation de l'eau par chauffage du support.

Cependant, ce moyen présente des inconvénients.

En raison de leur faible capacité de rétention de l'eau, ces systèmes ne peuvent être utilisés dans l'environnement qu'avec de faibles débits d'aspiration qui conduisent à des périodes de prélèvement de l'ordre d'une semaine. De plus, la récupération de l'eau tritiée pour mesure nécessite une installation encombrante et des manipulations importantes et délicates. Enfin ces supports sont à usage unique pour éviter toute contamination d'un échantillon sur l'autre. Dans les systèmes de prélèvements par pièges froids, on piège la vapeur d'eau tritiée sur une surface froide pour provoquer sa condensation. Il existe des dispositifs de prélèvements statiques où l'air n'est pas agité et des dispositifs de prélèvements dynamiques où l'air est agité.

Au cours des prélèvements statiques, le piège froid est constitué par une plaque d'acier inoxydable directement placée au contact de l'air et refroidie en permanence par de la carboglace ou de l'azote liquide. Le givre formé sur la surface est récupéré manuellement à l'aide d'une raclette.

Après réchauffage du givre, la concentration en tritium est mesurée dans l'eau collectée par une méthode connue en soi.

L'inconvénient de ces dispositifs est qu'ils ne sont pas automatisables, en raison du mode de refroidissement permanent de la surface. De plus, étant donné qu'il est impossible d'interrompre le refroidissement de la plaque, il peut y avoir contamination d'un échantillon sur l'autre.

Dans les systèmes dynamiques, contrairement aux systèmes statiques, le piège froid tel qu'un système à effet Peltier ou à azote liquide ou à liquide réfrigérant refroidi par des moyens de refroidissement comme dans le brevet JP11064532, n'est pas directement placé dans l'air, mais disposé dans une chambre. Une pompe fait passer l'air à analyser dans cette chambre où la vapeur se condense en continu. Dans le cas du système à effet Peltier qui ne peut être utilisé qu'avec une température d'air supérieure à 0°C, l'eau est collectée en permanence et la concentration en tritium peut alors être mesurée dans l'échantillon. Dans le système du brevet JP11064532, après arrêt des moyens de refroidissement et réchauffage du piège froid, la concentration en tritium est mesurée dans l'eau condensée récupérée. Ces dispositifs de prélèvements dynamiques par pièges froids présentent également des inconvénients.

En raison de l'utilisation d'une pompe, la quantité d'eau prélevée est limitée par le débit de la pompe et impose des temps de prélèvement plus importants qu'avec un système statique. Le système avec la source froide réalisée par effet Peltier ne peut être utilisé qu'avec une température d'air supérieure à 0°C, car il recueille l'eau en continu et, de plus, il peut exister un fractionnement isotopique de l'échantillon. Le système avec l'azote liquide comme source froide n'est pas automatisable, car la chambre de prélèvement plonge en permanence dans le réservoir d'azote liquide et la récupération de l'échantillon nécessite une extraction manuelle de la chambre. Enfin le système du brevet JP11064532 est compliqué à mettre en oeuvre, nécessitant par exemple l'installation de tout un réseau de canalisations susceptible d'être contaminé en tritium et de fausser les résultats.

Afin de remédier aux inconvénients liés aux moyens connus exposés ci-dessus, on a recherché un procédé automatique, de grande sensibilité, utilisable pour des températures de l'air pouvant être inférieures à 0°C et n'engendrant pas de contamination d'un échantillon à l'autre.

L'invention a pour objet un dispositif de prélèvement automatique du tritium dans la vapeur d'eau de l'air par piège froid. Le procédé réalisé avec le dispositif selon l'invention comprend une première étape de condensation de la vapeur d'eau de l'air par refroidissement sur un piège froid et une deuxième étape de récupération de la glace formée à l'étape précédente sous forme de liquide de condensation. L'air est contenu dans une enceinte de prélèvement et est mis en contact avec un piège froid porté à une température inférieure à 0°C et le liquide de condensation est obtenu par réchauffage du piège froid.

Le réchauffage est obtenu par arrêt du refroidissement produit par le piège froid.

L'arrêt du refroidissement produit par le piège froid est complété par un chauffage, lorsque la température extérieure est inférieure à 0°C.

De préférence, le piège froid est constitué par un serpentin relié à un réservoir de fluide de refroidissement.

Le piège froid peut aussi être couplé à un cryogénérateur.

De manière avantageuse, le piège froid est constitué par un serpentin à l'intérieur duquel circule de l'azote liquide pompé à partir d'un réservoir.

Préférentiellement, lorsqu'un chauffage du piège froid est nécessaire, il est obtenu par injection d'air chaud à l'intérieur du serpentin, après déconnexion du réservoir d'azote liquide.

Le liquide de condensation est recueilli par gravité dans un réceptacle disposé sous le piège froid.

De préférence, le dispositif selon l'invention comprend une enceinte de prélèvement d'air abritant un serpentin relié par une pompe à un réservoir d'azote liquide extérieur et un réceptacle disposé sous le serpentin.

Le procédé réalisé avec le dispositif selon l'invention présente les avantages ci-après :
- grande sensibilité, étant donné que le temps de prélèvement est court puisqu'il n'est pas limité par le débit d'une pompe et qu'il n'y a pas de dilution de l'échantillon,
- automatisation, étant donné qu'il n'y a pas d'intervention manuelle pour récupérer l'échantillon,
- possibilité d'utilisation pour des températures inférieures à 0°C,
- absence de contamination d'un prélèvement sur l'autre, étant donné que le serpentin est séché par évaporation d'un échantillon à l'autre,
- absence de fractionnement isotopique de l'échantillon.

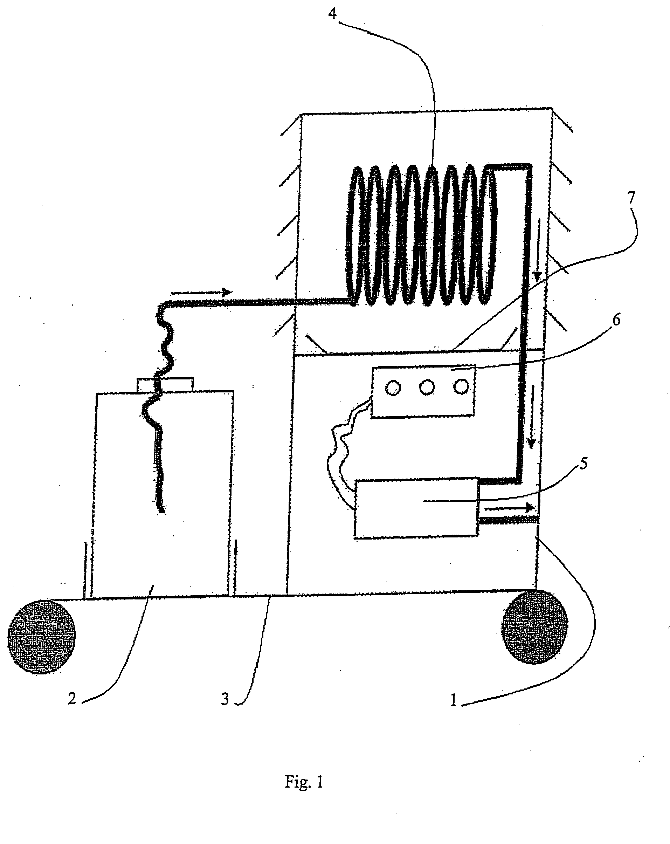

La description sera mieux comprise à l'aide des exemples non limitatifs ci-après illustrés par la figure 1.

La figure 1 est une vue schématique d'une installation de prélèvement d'air dans l'environnement pour la mise en oeuvre du procédé selon l'invention.

### Exemple de réalisation :

La figure 1 comporte une enceinte de prélèvement de type « abri météo » 1 réalisée en acier inoxydable et un réservoir d'azote liquide 2, le tout porté par un plateau roulant 3. L'enceinte de prélèvement 1 contient, dans sa partie haute, un serpentin 4 formé par un tube en cuivre composé de 14 spires non jointives et, dans sa partie basse, une pompe 5 et un tableau électrique 6 étanche à l'eau. Sous le serpentin est disposé un réceptacle 7 servant à recueillir l'eau condensée sur le serpentin.

L'ensemble de l'installation, qui présente une longueur de 1,55 m pour une largeur de 0,65 m et une hauteur de 1,75 m et pèse 192 kg est néanmoins facilement déplaçable grâce à son plateau roulant.

Pour réaliser un prélèvement, on démarre la pompe 5 afin d'évaporer l'azote liquide contenu dans le réservoir 2. En s'évaporant, l'azote liquide refroidit progressivement le serpentin 4 et l'eau se condense sur les spires du serpentin.

On arrête la pompe 5 au bout de 15 minutes. La glace formée sur les spires se réchauffe et est récupérée par gravité sous forme liquide dans le réceptacle 7. Si la température est trop basse pour permettre la fusion de la glace, on peut injecter de l'air chaud à l'intérieur du serpentin 4, après déconnexion de celui-ci du réservoir 2.

On récupère l'eau pour analyse, de manière connue, par scintillation liquide, afin de mesurer la concentration en tritium. Les quantités collectées varient en fonction des conditions météorologiques et sont, en moyenne, de l'ordre de 30 cm³.

Lors d'un prélèvement, la pression atmosphérique, la température et l'humidité de l'air sont mesurées par une sonde PTU avec une fréquence d'un Hertz. Ces mesures météorologiques permettent de convertir les résultats des mesures de tritium de l'unité Bq.L-1 d'eau prélevée en unité Bq.m-3 d'air.

### Comparaison du procédé de l'invention avec le procédé par barbotage :

Les performances du procédé selon l'invention ont été évaluées par comparaison avec le procédé par barbotage qui est le seul disponible sur le marché et dont le dispositif est commercialisé sous la dénomination HAG 7000.

### 1. Evaluation de la sensibilité :

Le procédé de l'invention permet de réaliser des prélèvements d'un volume minimal de 10 cm³ d'eau en 15 minutes, le prélèvement moyen étant de 30 cm³. Pour obtenir un volume de 10cm³ d'eau, ce qui représente 0,67 m³ d'air, dans le procédé par barbotage, on doit effectuer un prélèvement pendant 45 heures, le débit moyen du barboteur étant de 15L/h.

Le procédé selon l'invention permet donc un gain de temps d'un facteur 180.

De plus, il faut prendre en compte le facteur de dilution.

Le procédé par barbotage nécessite l'utilisation de plusieurs biberons de 180 cm³ d'eau des Abatilles. En considérant que la majeure partie du prélèvement se fait dans le premier biberon, le facteur de dilution pour un prélèvement de 45 heures est alors de 18 par rapport au procédé de l'invention où l'on collecte un volume de 10 cm³.

Ainsi, en considérant le temps de prélèvement et le facteur de dilution, le procédé de l'invention permet d'abaisser la limite de détection pour la mesure de la concentration en tritium d'au moins un facteur de 3000.

### 2. Evaluation vis-à-vis de la discrimination isotopique :

Deux expériences ont été réalisées afin d'évaluer les performances du procédé de l'invention vis-à-vis d'un éventuel problème de discrimination isotopique du tritium par rapport à l'hydrogène, car la discrimination isotopique peut biaiser la représentativité des mesures.

### a)Résultats obtenus par le procédé de l'invention avec de la vapeur d'eau tritiée de concentration connue :

On a généré, dans une enceinte close, de la vapeur d'eau de concentration connue en tritium, puis on a condensé cette vapeur d'eau sur le serpentin et on l'a collectée pour la mesure du tritium par scintillation liquide. Une comparaison a pu alors être effectuée entre la concentration en tritium dans la vapeur d'eau de concentration connue et la concentration en tritium de l'eau collectée.

Une eau marquée en tritium a été placée dans un réceptacle sous le serpentin de l'installation représentée sur la figure 1 pour permettre son évaporation naturellement, puis une aliquote a été prélevée pour mesure.

Un second réceptacle a été placé au-dessus du premier, afin de récupérer à la fin de l'expérience l'eau dont la concentration en tritium est à mesurer qui correspond à la vapeur d'eau condensée sur le serpentin. L'ensemble constitué par le serpentin et les deux réceptacles a été isolé de l'extérieur par un confinement d'un volume de 35 L en vinyle.

La mise en place effectuée, la pompe de l'installation a été démarrée, afin de réaliser le cycle d'évaporation de l'eau marquée en tritium, puis de condensation sur le serpentin. Après 15 minutes, la pompe a été arrêtée et les condensas récupérés dans le second réceptacle. La concentration en tritium dans l'eau condensée a été mesurée par scintillation liquide.

Pendant l'expérience, la pression, la température et l'humidité de l'air ont été mesurées, afin de tenir compte de la quantité de vapeur d'eau initialement présente dans le confinement de vinyle. L'expérience a été renouvelée 5 fois.

Les concentrations moyennes dans l'eau condensée et l'eau marquée en tritium sont respectivement de 252,8±15,1 Bq.L⁻¹ et 247,0±14,8 Bq.L⁻¹. Il y a donc un très bon accord entre ces deux valeurs. De plus, sur les 5 expériences, les concentrations en tritium dans l'eau condensée varient peu, puisqu'elles sont comprises entre 222,7±13,3 Bq.L⁻¹ et 262, 0±15,7 Bq.L⁻¹. Les concentrations en tritium dans l'eau marquée en tritium s'échelonnent entre 244,0± Bq.L⁻¹ et 257, 0± Bq.L⁻¹. Le rapport moyen entre les concentrations en tritium dans l'eau marquée en tritium et dans l'eau condensée est de 1,02.

L'analyse de ces résultats indique que le procédé selon l'invention permet la récupération de la totalité du tritium de la vapeur d'eau contenue dans la masse d'air dans laquelle il se trouve.

La discrimination isotopique entre le tritium et l'hydrogène, bien qu'elle existe physiquement, n'a donc pas d'influence sur les prélèvements réalisés par le procédé de l'invention.

### b)Résultats obtenus en utilisant le procédé de l'invention et le procédé par barbotage:

On a comparé les performances du procédé de l'invention avec celles du procédé par barbotage avec un barboteur de type HAG 7000 communément utilisé dans l'industrie nucléaire. A cette fin, un suivi de la concentration en tritium dans l'air pendant 4 périodes d'une semaine a été réalisé au niveau d'un bâtiment contenant les piscines de stockage de combustibles usés.

Pendant une période d'une semaine, un barboteur a prélevé en continu de la vapeur d'eau avec un débit de 20 L.h⁻¹.

La vapeur ainsi collectée a été analysée par scintillation liquide.

Pendant la même période, des prélèvements ponctuels pendant 15 minutes et journaliers ont été faits, de façon aléatoire, selon le procédé de l'invention. Chaque prélèvement a été analysé par scintillation liquide.

De plus, la pression, la température et l'hygrométrie de l'air ont été mesurées pendant cette période.

Les valeurs obtenues pendant les 4 campagnes d'une semaine chacune ont peu varié pendant chaque semaine et d'une semaine sur l'autre.

Les valeurs moyennes de concentration en tritium des semaines 1 à 4 sont respectivement de 0,50±0,03 Bq.m³, 0,46±0,03 Bq.m³, 0,38±0,02 Bq.m³ et 0,42±0,03 Bq.m³.

Pendant les mêmes semaines, les résultats obtenus par barbotage sont pour les semaines 1 à 4 respectivement de 0,49±0,03 Bq.m³, 0,54±0,03 Bq.m³, 0,41±0,02 Bq.m³ et 0,34±0,02 Bq.m³.

Le rapport moyen entre les résultats obtenus par le procédé de l'invention et le barboteur est de 1,004.

Il y a donc un très bon accord entre les résultats obtenus par le procédé de l'invention et ceux obtenus avec le barboteur de type HAG 7000.

Le procédé selon l'invention peut être utilisé pour réaliser un suivi de la concentration en tritium contenu dans la vapeur d'eau de l'air dans l'environnement, aussi bien que dans les installations nucléaires, telles que des bâtiments abritant des piscines de stockage de combustibles irradiés.

## Revendications

1. Dispositif de prélèvement automatique par piège froid (4) du tritium présent dans la vapeur d'eau de l'air comportant une enceinte (1)abritant un piège froid (4), des moyens (2, 5) de refroidissement de ce piège froid à une température inférieure à 0°C et des moyens pour démarrer et pour arrêter ces moyens de refroidissement du piège froid, dispositif **caractérisé en ce que** :
- l'enceinte (1) est de type abri météo,
- un réceptacle (7) est disposé sous le piège froid.

2. Dispositif de prélèvement selon la revendication 1, **caractérisé en ce que** le piège froid (4) est constitué par un serpentin.

3. Dispositif de prélèvement selon l'une quelconque des revendications 1 et 2, **caractérisé en ce que** les moyens (2, 5) de refroidissement du piège froid (4) sont constitués par un cryogénérateur.

4. Dispositif de prélèvement selon la revendication 2, **caractérisé en ce que** les moyens de refroidissement du piège froid sont constitués par une pompe (5) et par un réservoir de fluide de refroidissement (2) tout deux reliés audit serpentin (4), le fluide de refroidissement étant apte à circuler à l'intérieur du serpentin lorsque la pompe fonctionne et à le porter à une température inférieure à 0°C.

5. Dispositif de prélèvement selon la revendication 4, **caractérisé en ce que** le fluide de refroidissement est constitué par de l'azote liquide (2).

6. Dispositif de prélèvement selon la revendication 5, **caractérisé en ce qu'**il comporte des moyens d'injection d'air chaud dans le serpentin après déconnexion du réservoir d'azote liquide.

7. Dispositif de prélèvement selon la revendication 5, **caractérisé en ce que** l'enceinte et le réservoir d'azote sont placés sur un plateau roulant (3).

## Patentansprüche

1. Vorrichtung für die automatische Entnahme von Tritium aus Wasserdampf in der Luft mittels Kühlfalle (4) mit einem abgeschlossenen Bereich (1), der eine Kühlfalle (4) enthält, Mitteln (2, 5) zum Abkühlen dieser Kühlfalle auf eine Temperatur unter 0°C und Mittel, um diese Kühlmittel der Kühlfalle zu starten und anzuhalten, wobei die Vorrichtung **dadurch gekennzeichnet ist, dass**:
- der abgeschlossene Bereich (1) von der Art eines Wetterhauses ist,
- ein Behälter (7) unter der Kühlfalle angeordnet ist.

2. Entnahmevorrichtung nach Anspruch 1, **gekennzeichnet dadurch, dass** die Kühlfalle (4) aus einer Spule besteht.

3. Entnahmevorrichtung nach einem beliebigen der Patentansprüche 1 und 2, **gekennzeichnet dadurch, dass** die Mittel (2, 5) zum Kühlen der Kühlfalle (4) aus einem Kryogenerator bestehen.

4. Entnahmevorrichtung nach Anspruch 2, **gekennzeichnet dadurch, dass** die Kühlmittel der Kühlfalle aus einer Pumpe (5) und einem Kühlflüssigkeitstank (2) bestehen, die beide mit der Spule (4) verbunden sind, wobei die Kühlflüssigkeit in der Spule umlaufen kann, wenn die Pumpe in Betrieb ist, und sie auf eine Temperatur unter 0°C bringen kann.

5. Entnahmevorrichtung nach Anspruch 4, **gekennzeichnet dadurch, dass** die Kühlflüssigkeit aus flüssigem Stickstoff (2) besteht.

6. Entnahmevorrichtung nach Anspruch 5, **gekennzeichnet dadurch, dass** sie Mittel zur Zufuhr von heißer Luft in die Spule nach Abkoppelung des Flüssigstickstofftanks umfasst.

7. Entnahmevorrichtung nach Anspruch 5, **gekennzeichnet dadurch, dass** der abgeschlossene Bereich und der Stickstofftank auf einem Rollwagen (3) platziert sind.

## Claims

1. A device for automatically sampling tritrium present in the water vapor in the air by cold trap (4), comprising an enclosure (1) accomodating a cold trap (4), means (2, 5) for cooling said cold trap at a temperature lower than 0°C, and means for actuating and stopping said means for cooling the cold trap, the device being **characterized in that**:
- the enclosure (1) is of wheather screen type,
- a receptacle (7) is arranged under the cold trap.

2. The sampling device according to claim 1, **characterized in that** the cold trap (4) is constituted by a coil.

3. The sampling device according to any of claims 1 and 2, **characterized in that** the means (2, 5) for cooling the cold trap (4) are constituted by a cryogenerator.

4. The sampling device according to claim 2, **characterized in that** the means for cooling the cold trap are constituted by a pump (5) and a refrigerant tank (2) both connected to said coil (4), the refrigerant being adapted to flow inside the coil when the pump operates and to bring it at a temperature lower than 0°C.

5. The sampling device according to claim 4, **characterized in that** the refrigerant is constituted by liquid nitrogen (2).

6. The sampling device according to claim 5, **characterized in that** it comprises means for injecting hot air inside the coil after separation from the liquid nitrogen tank.

7. The sampling device according to claim 5, **characterized in that** the enclosure and the nitrogen tank are arranged on a dolly (3).
